(19) **Europäisches Patentamt / European Patent Office / Office européen des brevets**

(11) **EP 1 711 071 B1**

(12) **FASCICULE DE BREVET EUROPEEN**

(45) Date de publication et mention
de la délivrance du brevet:
**29.08.2007 Bulletin 2007/35**

(21) Numéro de dépôt: **05717368.4**

(22) Date de dépôt: **06.01.2005**

(51) Int Cl.:
*A23L 1/30* (2006.01)    *A23L 1/29* (2006.01)

(86) Numéro de dépôt international:
**PCT/FR2005/000020**

(87) Numéro de publication internationale:
**WO 2005/067736 (28.07.2005 Gazette 2005/30)**

(54) **UTILISATION DE FIBRES VEGETALES DE GRANULOMETRIE TRES FINE POUR LA PREPARATION D UNE COMPOSITION ALIMENTAIRE DESTINEE A DIMINUER LA BIODISPONIBILITE DES MYCOTOXINES**

VERWENDUNG VON PFLANZENFASERN MIT FEINER KORNGRÖSSE FÜR DIE HERSTELLUNG EINER NÄHRSTOFFZUSAMMENSETZUNG ZUR VERRINGERUNG DER BIOLOGISCHEN VERFÜGBARKEIT VON MYKOTOXINEN

USE OF VEGETABLE FINE GRAIN SIZED FIBRES FOR PREPARING A NUTRITIONAL COMPOSITION FOR REDUCING MYCOTOXIN BIOAVAILABILITY

(84) Etats contractants désignés:
**AT BE BG CH CY CZ DE DK EE ES FI FR GB GR HU IE IS IT LI LT LU MC NL PL PT RO SE SI SK TR**

(30) Priorité: **07.01.2004 FR 0400090**

(43) Date de publication de la demande:
**18.10.2006 Bulletin 2006/42**

(73) Titulaire: **Realdyme**
**28700 Garancières en Beauce (FR)**

(72) Inventeurs:
• **TANGNI, Emmanuel, Kossi**
**B-1348 Louvain-La-Neuve (BE)**
• **LARONDELLE, Yvan**
**B-1030 Bruxelles (BE)**
• **De MEEÜS d'ARGENTEUIL, Ludovic**
**B-1450 Chastre (BE)**
• **AOUDIA, Nabilla**
**ALGER (DZ)**

(74) Mandataire: **Goulard, Sophie**
**Cabinet ORES**
**36, rue de St Pétersbourg**
**75008 Paris Cedex (FR)**

(56) Documents cités:
**US-A- 4 770 880**

• SMITH T K: "Influence of dietary fiber, protein and zeolite on zearalenone toxicosis in rats and swine" JOURNAL OF ANIMAL SCIENCE, NEW YORK, NY, US, vol. 50, no. 2, 1980, pages 278-285, XP002117553 ISSN: 0021-8812
• CARSON MS, SMITH TK: "Effect of feeding alfalfa and refined plant fibers on the toxicity and metabolism of T-2 toxin in rats" JOURNAL OF NUTRITION, vol. 113, 1983, pages 304-313, XP009035132
• FRAPE DL, WAYMAN BJ, TUCK MG, JONES E: "The effects of gum arabic, wheat offal and various of its fractions on the metabolism of 14C-labelled aflatoxin B1 in the male weanling rat" BRITISH JOURNAL OF NUTRITION, vol. 48, 1982, pages 97-110, XP009035131 UK

**Description**

La présente Invention est relative à l'utilisation de fibres végétales de granulométrie très fine pour la préparation d'une composition alimentaire destinée à diminuer la biodisponibilité des mycotoxines.

L'importance de l'innocuité des aliments pour la sécurité alimentaire a été largement reconnue, en particulier par les divers gouvernements qui ont participé en 1992 à la Conférence Internationale sur la Nutrition *("International Conference on Nutrition")* qui s'est déroulée à Rome (Italie) et en 1996 au Sommet Mondial sur l'Alimentation *("World Food Summit")* de Rome (Italie).

La qualité et la sécurité des aliments peuvent être menacées par un grand nombre de facteurs, y compris par la présence de toxines naturelles. En effet, parmi la longue liste de toxines pouvant naturellement se retrouver dans les produits d'alimentation courante, les mycotoxines représentent une catégorie très importante qui figure parmi les plus étudiées dans la mesure où leur ubiquité et leurs effets néfastes sur la santé humaine et animale provoquent une inquiétude générale (FAO, 1999, *"Preventing Mycotoxin contamination",* Publication n°23, Rome, Italie, p 55).

Les produits agricoles sont les cibles potentielles de ravageurs et de maladies. Ils portent une flore microbienne variable et nombreuse comprenant principalement des bactéries, des levures et des champignons filamenteux. Leur présence peut notamment provoquer une détérioration de la qualité des produits agricoles, allant parfois jusqu'à leur destruction pure et simple. Parmi ces microorganismes, certains champignons filamenteux sont responsables de la production des mycotoxines, qui s'observe au cours de la croissance des produits agricoles dans les champs ou bien au cours de leur stockage dans des conditions propices d'humidité et de température. Les principaux genres de champignons *(fungi)* producteurs de mycotoxines sont *Penicillium, Fusarium, Aspergillus* et *Alternaria.*

Les mycotoxines sont des métabolites secondaires de composition chimique très variable, mais en général de faible poids moléculaire. Leurs effets néfastes sur la santé humaine, qu'ils soient aigus ou chroniques, sont aussi très variés. Leurs cibles sont principalement les reins, le foie, le tractus gastro-intestinal et les systèmes nerveux et immunitaires. A ce jour, environ cinq cents mycotoxines ont été découvertes et leur nombre ne cesse d'augmenter au fur et à mesure que la recherche avance. Toutefois, seule une vingtaine sont bien identifiées comme une menace réelle pour la sécurité alimentaire. Parmi les différentes familles de mycotoxines rencontrées dans les produits alimentaires, on peut notamment citer les Aflatoxines (AFLA), les toxines de l'ergot, les Fumonisines (FB), l'Ochratoxine A (OTA), la Patuline (PAT), la Stérigmatocystine, la Zéaralénone (ZEA) et les Trichotécènes dont le Déoxynivalénol (DON), En fonction de leur nature, ces mycotoxines peuvent avoir des effets néfastes et divers sur la santé humaine ou animale ; elles peuvent notamment être hépato et immunotoxiques, carcinogènes, tératogènes, neurotoxiques, néphrotoxiques, ou bien encore entraîner des troubles digestifs ou des hémorragies.

Les principales denrées alimentaires susceptibles d'être contaminées par les mycotoxines sont les céréales, les noix, les fruits secs, le café, le cacao, les épices, les graines oléagineuses, les pois et les fèves, ainsi que les fruits. Leurs produits dérivés peuvent donc être contaminés, suivant la stabilité de la toxine au cours du processus de transformation. Il en résulte que ces mycotoxines, et notamment l'OTA, peuvent être transmises à de nombreux produits de consommation animale ou humaine tels que les farines animales, le vin, la bière, le pain et les produits dérivés du café et du cacao (Abarca ML. et al., J. Food Prot., 2001, 64(6), 903-906 ; Walker R., Adv. Exp. Med. Biol., 2002, 504, 249-255). Il existe également un risque non négligeable de contamination secondaire *via* certaines denrées d'origine animale comme la viande et les abats d'animaux monogastriques (Pittet A., Rev. Méd. Vét., 1998, 149, 479-492).

Les mycotoxines sont des composés très stables et résistants à la majorité des processus de transformation des produits agro-alimentaires. Par conséquent, et compte tenu de leurs effets néfastes sur la santé, il est de la plus grande importance de pouvoir disposer de moyens efficaces permettant :

-    soit de prévenir la contamination des produits alimentaires,
-    soit de les décontaminer avant et/ou après leur transformation.

La première approche n'est pas toujours réalisable compte tenu des conditions de culture ou de stockage des matières premières.

La deuxième approche doit donc être mise en oeuvre au niveau industriel et divers procédés physico-chimiques ou biologiques ont déjà été proposés dans ce sens. Ces procédés de décontamination peuvent être classés en trois grandes catégories :

1) la première catégorie consiste à dégrader les toxines en des produits moins toxiques ou non toxiques afin que leur ingestion soit moins préjudiciable à l'organisme ;
2) la deuxième catégorie consiste à soumettre l'aliment à une étape de microfiltration. A titre d'exemple, on peut notamment citer le brevet américain n° 5,248,382 qui décrit une méthode pour réduire le taux de mycotoxines dans les jus de fruits, et en particulier le taux de patuline, au moyen d'une filtration sur une résine microporeuse capable de retenir la patuline par chémisorption et dans laquelle le diamètre des pores est inférieur à 20 Angström. Bien qu'efficace, cette méthode présente l'inconvénient de mettre en oeuvre un matériel spécifique et coûteux et de n'être applicable qu'à des produits alimentaires se présentant sous une forme liquide ;

3) la troisième catégorie consiste à utiliser des adsorbants afin de retenir, au moins en partie, les mycotoxines. Ces adsorbants sont soit ajoutés au cours de la fabrication des produits alimentaires puis éliminés de ceux-ci avant consommation (généralement par filtration) afin de réduire la teneur en mycotoxines dans l'aliment final ; soit ajoutés aux aliments consommés pour diminuer la biodisponibilité des mycotoxines lors de la digestion.

Dans les nombreux documents de l'art antérieur décrivant des procédés appartenant à cette troisième catégorie, les mycotoxines sont éliminées par l'action d'adsorbants non biologiques, généralement inorganiques.

Parmi de tels adsorbants inorganiques, il a notamment déjà été proposé d'utiliser :

- les aluminosilicates sodiocalciques hydratés (HSCAS) qui sont capables d'adsorber des aflatoxines présentes dans un milieu. Cependant leur effet est moindre vis-à-vis de l'OTA (Huwig A. et al., Tox. Letters, 2001, 122, 179-188) ;
- les phyllosilicates (Diaz D. E. et al., J. Dairy Sci., 1999, 82 (suppl. 1), 838 ;
- les charbons actifs pour lesquels des tests *in vivo* montrent un effet préventif vis-à-vis des aflatoxicoses et une augmentation des performances des animaux lorsqu'ils sont nourris avec des aliments contaminés (Galvano et al., J. Food Prot., 1997, 60, 985-991) de même qu'une diminution de la teneur en OTA dans les tissus, le sang et la bile des animaux suite à l'incorporation de l'adsorbant dans les régimes (Ramos A. J. et Hemandez E., Animal Feed Science and Technology, 1997, 62, 263-269 ; Huwig *et al.,* 2001, pré-cité) ;
- les résines telles que la cholestyramine et la polyvinylpyrrolidone qui sont également capables de fixer l'OTA (Piva A. et Galvano F., 1999, "Nutritional approaches to reduce the impact of mycotoxins", Proceedings of Alltech's 15th Annual Symposium, T. P. Lyons et K.A. Jacques (eds.), 381-400, Nottingham University Press, Nottingham, UK).

En revanche, les études *in vivo* ne révèlent pas une protection systématique de ces adsorbants vis-à-vis de la toxicité des mycotoxines et en particulier de l'OTA. En effet, l'étude réalisée par Bauer J. (Monatsh Veterinarmed, 1994, 49, 175-181) n'a démontré aucune réduction de la concentration sanguine et tissulaire de l'OTA chez des pigeons, lorsque l'ingestion d'aliments contaminés par cette toxine était associée à la prise de bentonite (demande de brevet allemand DE 3 810 004), de HSCAS, de cholestyramine ou de parois de levures. Les mêmes observations ont été faites par Scheideler S. E. (Poultry Science, 1993, 72, 282-288) et Ledoux D. R. et al., 2001 ("In vitro binding mycotoxins by adsorbents does not always translate into in vivo efficacy.", In : Mycotoxins and phycotoxins in perspective at the turn of the millennium. Proceedings of the Xth International IUPAC Symposium in Mycotoxins and Phycotoxins, édité par W. J. de Koe, R. A. Samson, H. P. Van Egmond, J. Gilbert et M. Sabino, Wageningen, The Netherlands, 279-287). Plus récemment, Santin E. et al. (J. Appl. Poultry Res., 2002, 11(1), 22-28) ont démontré que l'addition de HSCAS à la dose de 0,25 % n'améliore guère l'impact négatif de l'OTA (2 ppm) sur les paramètres physiologiques chez des poulets.

Par ailleurs, les ligands mentionnés ci-dessus peuvent occasionner des pertes d'éléments nutritifs et des modifications de la qualité organoleptique des aliments.

De plus, il reste que l'élimination totale des mycotoxines est impossible. C'est pourquoi il semble important de compléter le panel des mesures préventives et correctives exposées précédemment par l'emploi d'agents inoffensifs pour l'organisme et acceptables d'un point de vue organoleptique, qui soient capables de lier les mycotoxines pour en réduire la biodisponibilité dans l'organisme après ingestion d'un aliment contaminé.

Les Inventeurs se sont donnés pour but de pourvoir à une méthode biologique de détoxication de milieux alimentaires permettant de remédier à l'ensemble de ces inconvénients, et qui conduit à une diminution de la biodisponibilité des mycotoxines après ingestion de produits alimentaires contaminés.

A cette occasion, les Inventeurs ont mis en évidence, de façon surprenante, que la capacité d'adsorption de fibres végétales essentiellement insolubles vis-à-vis des mycotoxines est grandement améliorée lorsque l'on sélectionne des fibres végétales se présentant sous la formes de microparticules dont au moins 90 % en poids ont une taille inférieure ou égale à 700 $\mu$m et peut être mise à profit afin de préparer une composition alimentaire capable de diminuer de façon significative la biodisponibilité des mycotoxines susceptibles d'être présentes dans les produits alimentaires ingérés.

L'utilisation de fibres alimentaires pour réduire l'absorption de mycotoxines est aussi connue par US 4 770 880; Carson et Smith, J. Nutr. 1983; 113 : 304-313 et Smith, J .Anim. Sci. 1980; 90 : 278-285.

La présente Invention a donc pour objet l'utilisation de fibres végétales micronisées essentiellement insolubles se présentant sous la formes de microparticules dont au moins 90 % en poids présentent une taille inférieure à 700 $\mu$m à titre d'ingrédient dans la préparation d'une composition alimentaire destinée à diminuer la biodisponibilité des mycotoxines chez l'homme ou l'animal lors de l'ingestion d'un aliment susceptible d'être contaminé par lesdites mycotoxines.

Les Inventeurs ont en effet observé que la capacité d'adsorption *in vitro* de ces fibres végétales vis-à-vis des mycotoxines se maintient *in vivo* et permet de diminuer la biodisponibilité et par voie de conséquence les effets néfastes des mycotoxines après ingestion dans l'organisme. Les Inventeurs ont par ailleurs constaté, lors d'expériences réalisées *in vitro,* que l'utilisation de fibres végétales répondant à ces caractéristiques en terme de granulométrie permettait d'augmenter de façon significative la capacité d'adsorption des fibres végétales vis-à-vis des mycotoxines par rapport aux fibres végétales ayant une taille supérieure à 700 μm.

Selon l'Invention, on entend par "essentiellement insoluble", une composition dans laquelle la fraction soluble des fibres (déterminée par analyse enzymatique) ne dépasse pas 55 % de la teneur en fibre totale. Par ailleurs, dans la description détaillée qui va suivre et sauf indication contraire de façon explicite, les termes "fibres végétales" font référence aux fibres végétales essentiellement insolubles ayant les caractéristiques de granulométrie telles que définies ci-dessus utilisables dans le cadre de l'Invention.

Selon une forme de réalisation avantageuse de la présente Invention, les fibres végétales utilisables se présentent de préférence sous la forme de microparticules dont 90 % environ en poids présentent une taille inférieure ou égale à 400 μm environ, et encore plus préférentiellement comprise entre environ 2 μm et 200 μm inclusivement ; la granulométrie étant mesurée par tamisage sur un tamis A 200 LS Air Jet Sieve commercialisé par la société Alpine (Augsburg, Allemagne), les fibres étant soumises à une dépression d'air de 250 à 700 mm de colonne d'eau pendant 6 minutes.

Les fibres végétales utilisées dans la cadre de l'invention peuvent notamment être préparées par micronisation selon le procédé décrit par exemple dans la demande de brevet FR 2 433 910. Les microparticules de fibres végétales utilisables selon l'Invention ont une forme sphérique ou sensiblement sphérique.

Selon une forme de réalisation avantageuse de l'Invention, ladite composition alimentaire est plus particulièrement destinée à diminuer la biodisponibilité des mycotoxines hydrophobes, les Inventeurs ayant en effet constaté *in vitro* que les mycotoxines hydrophobes ont une plus grande affinité pour les fibres végétales que les mycotoxines hydrophiles. A ce titre, les Aflatoxines et l'Ochratoxine A sont des exemples de mycotoxines hydrophobes.

Selon une forme de réalisation de l'Invention, les fibres végétales sont choisies parmi les fibres issues :

- de végétaux alimentaires choisis parmi les céréales, les légumineuses, les plantes potagères, les fruits y compris tropicaux, et plus généralement toute plante utilisée à des fins alimentaires,
- de végétaux utilisés par l'industrie du papier tels que les arbres, la canne à sucre, le bambou et la paille de céréale.

Parmi les fibres végétales issues de céréales, on peut en particulier citer les fibres de blé, d'orge, d'avoine, de maïs, de millet, de riz, de seigle, de sorgho, et leurs équivalents maltés.

Selon l'invention, on entend par équivalent malté les grains germés dont la germination a été stoppée par un traitement thermique et qui ont ensuite été débarrassés de leurs germes.

Parmi les fibres issues de végétaux alimentaires autres que les céréales, on peut notamment citer les fibres issues des pommes, des poires, des grains de raisins, des graines de lupin et de soja, des tomates, des pois, du café, etc.

Parmi ces fibres végétales, on préfère tout particulièrement :

- les fibres de blé micronisées vendues sous les dénominations commerciales Realdyme® et Realdyme® M, ce dernier se présentant sous la forme de microparticules dont au moins 90 % en poids ont une taille inférieure ou égale à 100 μm ;
- les fibres d'avoine micronisées vendues sous la dénomination commerciale Realdyme® A ;
- les fibres d'orges micronisées vendues sous la dénomination commerciale Realdyme ® O, et en particulier le Realdyme ® OF et le Realdyme ® OM ;
- les fibres de pomme micronisées vendues sous la dénomination Realdyme® P.

Toutes ces fibres sont disponibles auprès de la société REALDYME (Garancières en Beauce, La Haute Epine, France).

La nature des fibres utilisées conformément à l'Invention est de préférence choisie en fonction de la nature de la ou des mycotoxines susceptibles d'être présentes dans le milieu alimentaire et dont on veut diminuer la biodisponibilité après ingestion.

Ainsi, lorsque la composition alimentaire est principalement destinée à diminuer la biodisponibilité de l'Ochratoxine A, des aflatoxines, de la Fumonisine et/ou du déoxynivalénol, alors les fibres végétales seront préférentiellement choisies parmi les fibres de blé micronisées telles que les produits Realdyme® et Realdyme® M, les fibres d'avoine micronisées telles que par exemple le produit Realdyme® A et leurs mélanges. Selon l'Invention, et en ce qui concerne la diminution de la biodisponibilité de l'Ochratoxine A, on préfère tout particulièrement utiliser du Realdyme® M.

La composition alimentaire utilisable selon l'Invention est destinée aussi bien à l'alimentation animale qu'à l'alimentation humaine.

La composition alimentaire conforme à l'Invention peut se présenter sous plusieurs formes telles qu'un complément alimentaire, un ingrédient alimentaire (ou produit alimentaire intermédiaire : PAI) ou une matière première.

Lorsque la composition alimentaire conforme à l'invention se présente sous la forme d'un complément alimentaire, alors la quantité de fibres végétales au sein dudit complément peut représenter jusqu'à 100 % en poids du poids total dudit complément ; cette quantité étant préférentiellement comprise entre 80 et 100 % en poids.

Lorsque la composition alimentaire se présente sous la forme d'un PAI, elle est alors de préférence destinée à l'alimentation humaine et est utilisée à titre d'ingrédient au cours de la fabrication d'un produit alimentaire susceptible d'être contaminé par des mycotoxines. Dans ce cas, la composition alimentaire représente alors de 0,05 à 20 % en poids et encore plus préférentiellement de 0,1 à 5 % en poids par rapport au poids total du produit alimentaire fini.

Lorsque la composition alimentaire se présente sous la forme d'une matière première, elle est de préférence destinée à l'alimentation animale. La composition alimentaire peut alors être ajoutée à la ration alimentaire journalière qui est donnée aux animaux domestiques ou d'élevage et qui est susceptible d'être contaminée par des mycotoxines, et ce avant ingestion de ladite ration. Elle peut également être utilisée à titre de matière première ou d'additif au cours de la fabrication d'un aliment complet pour animaux domestiques ou d'élevage. Dans ces deux derniers cas, la composition alimentaire représente alors de préférence de 0,05 à 10 % en poids et encore plus préférentiellement de 0,3 à 2 % en poids par rapport au poids total de la ration alimentaire ou de l'aliment complet.

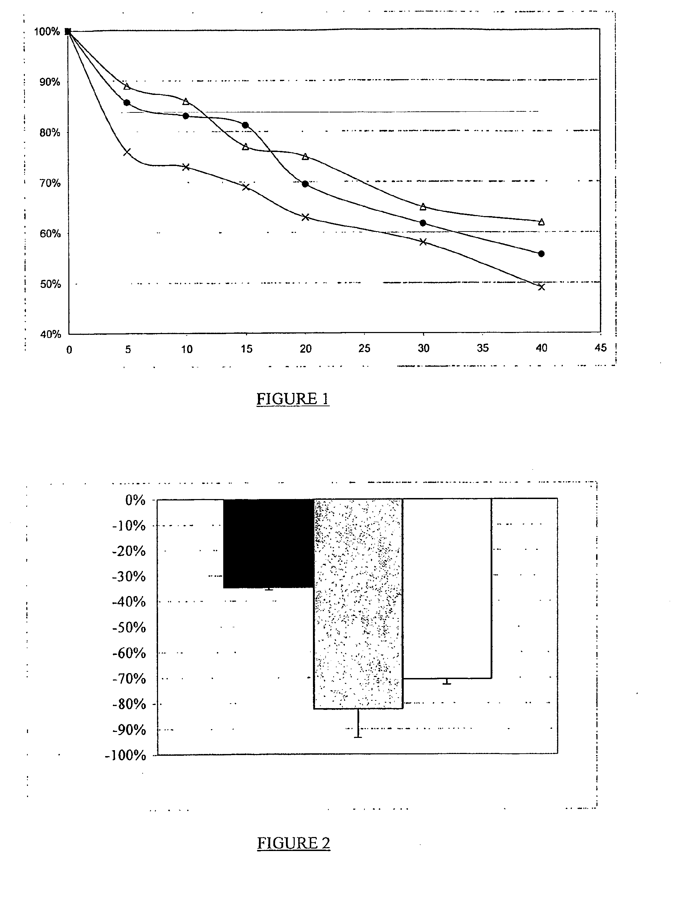

Outre les dispositions qui précèdent, l'Invention comprend encore d'autres dispositions qui ressortiront de la description qui va suivre, qui se réfère à un exemple de mise en évidence de la capacité d'adsorption d'OTA par des fibres végétales insolubles et criblage préliminaire de différentes fibres végétales, à un exemple d'étude de l'effet des variations de pH sur l'adsorption d'OTA dans un milieu liquide modèle, à un exemple de mise en évidence de l'adsorption des aflatoxines B1 par des fibres végétales insolubles dans un milieu liquide modèle, à une étude comparative de l'effet dose-réponse de l'adsorption de l'AFB1 par des fibres de blé micronisées et non micronisées et à un exemple d'étude de l'effet de l'incorporation de fibres de blé micronisées dans un régime alimentaire contaminé par de l'OTA donné à des rats sur la diminution de la biodisponibilité de l'OTA, ainsi qu'aux figures 1 à 5 annexées dans lesquelles :

- la figure 1 représente l'évolution de l'adsorption d'OTA, exprimée en pourcentage résiduel par rapport à la quantité présente initialement, par trois fibres différentes (Realdyme® M : croix ; Realdyme® OF : ronds pleins et Realdyme® OM : triangles vides) en milieu liquide modèle contenant une concentration initiale de 30 ng d'OTA/ml pour un volume initial de 25 ml et une durée de mise en contact de 45 minutes en fonction de la quantité de fibres en grammes par litre de milieu ;
- la figure 2 représente l'évolution de l'adsorption d'OTA (diminution de la quantité d'OTA en pourcentage de la quantité initialement présente) dans un milieu liquide modèle par des fibres Realdyme® M en fonction des variations de pH : barres noires : pH = 6 ; barres grises : pH = 2,2 ; barres blanches : après la remontée du pH de 2,2 à 4,8 ;

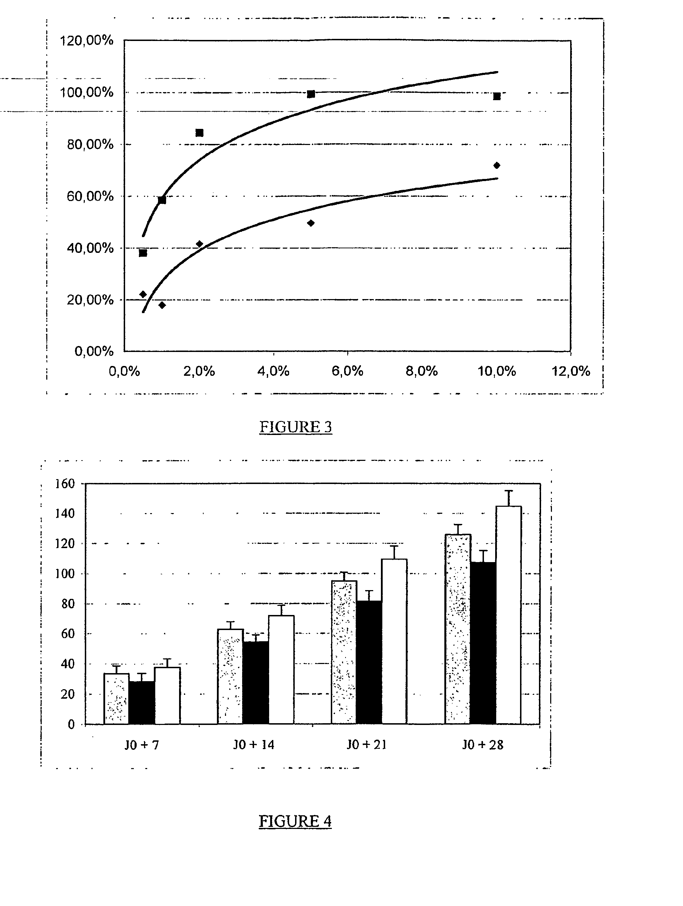

- la figure 3 représente la quantité d'AFB1 adsorbée dans un milieu PBS ajusté à pH 3 et initialement contaminé par de l'AFB1, cette quantité étant exprimée en pourcentage de la quantité d'AFB1 initialement présente dans ledit milieu ; carrés noirs : fibres de blé micronisées et losanges noirs : fibres de blé non micronisées ;
- la figure 4 représente la quantité cumulée de matières fécales éliminées (en g) en fonction du temps (exprimé en jours après le début de l'expérience : J0) chez des rats ayant reçu différents régimes alimentaires contaminés ou non en OTA et supplémentés ou non par des fibres de blé micronisées (barres grises : régime 1 = ration alimentaire non contaminée par de l'OTA ; barres noires : régime 2 = ration alimentaire contaminée par de l'OTA et barres blanches : régime 3 = ration alimentaire contaminée par de l'OTA et supplémentée par des fibres de blé micronisées) ;

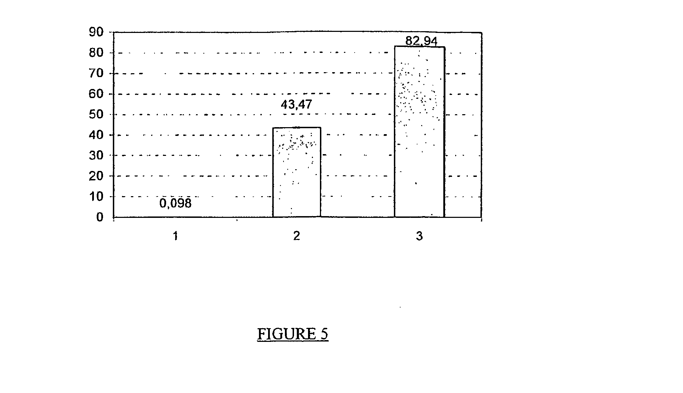

- la figure 5 représente la quantité d'OTA dans les fèces des rats (en $\mu$g) récoltés la quatrième semaine en fonction des différents régimes énoncés ci-dessus pour la description de la figure 4.

### EXEMPLE 1 : MISE EN EVIDENCE DE LA CAPACITE D'ADSORPTION D'OTA PAR DES FIBRES VEGETALES ET CRIBLAGE PRELIMINAIRE DE DIFFERENTES FIBRES VEGETALES

De façon surprenante, les Inventeurs ont mis en évidence que l'incorporation de fibres végétales micronisées dans un milieu liquide modèle permet, par l'adsorption de l'OTA sur les fibres, de diminuer la quantité de mycotoxines disponible dans ce milieu. Les tests *in vitro* reportés dans cet exemple ont été réalisés afin de démontrer la capacité d'adsorption de fibres végétales micronisées lorsqu'elles sont incorporées dans un milieu liquide contaminé en OTA et afin de déterminer quel est le temps de mise en contact nécessaire à une adsorption optimale de l'OTA par ces fibres. Ensuite, un criblage de trois fibres végétales différentes a été réalisé afin de déterminer quelles étaient les fibres les plus performantes vis-à-vis de l'adsorption de l'OTA. La capacité d'adsorption du Realdyme ® M a par ailleurs été comparée à celle de la matière première non micronisée (fibres de blé non micronisées).

### 1) Protocole expérimental

Une quantité déterminée de fibres végétales (environ 20 g/l) est mélangée dans un tube stérile de 50 ml avec 25 ml d'une solution aqueuse renfermant 2 % de Dextrose (vendu sous la dénomination commerciale D(+) glucose monohydraté par la société Merck), 5 % d'extrait de levures (vendu sous la dénomination commerciale Extrait de levures en Poudre par la société ICN Biomedical) et 1 % de peptone (vendu sous la dénomination Peptone par la société Duchefa), préalablement stérilisée à 121 °C pendant 15 minutes. Cette solution aqueuse présente un pH compris entre 6,0 et 6,2 et est dénommée "DYP" dans ce qui suit (milieu modèle). La solution DYP est ensuite contaminée par une quantité variable d'une solution d'OTA dans l'éthanol. La concentration d'OTA dans le milieu liquide modèle est de 57 ng/ml. Le contenu du tube est ensuite homogénéisé par agitation manuelle pendant 30 secondes, puis le tube est placé à agiter à 90 tours par minute (rpm) dans une chambre thermostatée à 25°C pendant 45 minutes. Un traitement contrôle sans adsorbant (témoin), c'est-à-dire sans fibre végétale, est effectué pour chaque expérience, et chacune de ces expériences est réalisée trois fois.

La suspension est ensuite centrifugée à 1830 g pendant 10 minutes à une température de 25°C puis le culot est séparé du surnageant. On extrait ensuite 1 ml de surnageant dans un tube stérile par 9 ml d'une solution de méthanol : eau (50 : 50 ; v/v). Le tube est ensuite vortexé pendant 30 secondes, puis centrifugé pendant 10 minutes à 820g à une température comprise entre 5 et 10°C. Cet extrait est ensuite dilué, filtré et analysé par chromatographie liquide haute performance (HPLC).

Le système HPLC consiste en une pompe à régime isocratique Perkin Elmer® LC049 vendue par la société Norwalk (USA) avec une boucle d'injection de 50 $\mu$l vendue sous la dénomination Rheodyne® par la société Cotati (USA), équipée d'une colonne $C_{18}$ d'une longueur de 150 mm et d'un diamètre de 4 mm vendue sous la dénomination Hypersil® BDS, d'une porosité de 3 $\mu$m, vendue par la société Tracer Analytica (Espagne), d'un détecteur à fluorescence RF 551 vendu par la société Shimadzu (Japon) muni d'une lampe xénon d'une puissance de 150 W réglé à une longueur d'onde d'excitation ($\lambda_{excitation}$) de 332 nm et à une longueur d'onde d'émission ($\lambda_{émission}$) de 462 nm, d'un intégrateur SP4290 vendu par la société Spectra Physics (USA). La phase mobile est composée d'un mélange acétonitrile/eau/ acide acétique (450/540110 ; v/v) filtré sur membrane de 0,25 $\mu$m et dégazée à l'hélium pendant 15 minutes. Le débit de la phase liquide est fixé à 1 ml/min à une pression comprise entre 2900 et 3000 psi.

La quantité d'OTA totale adsorbée est obtenue par différence entre la quantité initiale et finale présente dans le surnageant.

Dans cet exemple, les fibres végétales micronisées suivantes ont été utilisées : Realdyme® M, Realdyme® OF et Realdyme® OM, et ce à différents dosages.

## 2) Résultats

Les résultats obtenus sont reportés dans les tableaux I à IV ci-après ainsi que sur la figure 1 annexée.

Les pourcentages d'OTA adsorbées sur les fibres Realdyme® M en fonction de la durée de la mise en contact, avec le milieu modèle contenant 57 $\mu$g/l d'OTA, à un pH compris entre 6,0 et 6,2 sont reportés dans le Tableau I ci-après :

**TABLEAU I**

| | Quantité de mycotoxines adsorbée (%) | | |
|---|---|---|---|
| | Durée de la période de mise en contact (heures) | | |
| Quantité de Realdyme® M (g/l) | 3 | 24 | 48 |
| 0 (Contrôle) | 0 | 0 | 0 |
| 10 | 46,7 | 52,5 | 49,8 |
| 16 | 59,8 | 65,3 | 61,6 |
| 20 | 68,9 | 69,7 | 71,7 |
| 30 | 68,0 | 72,3 | 73,6 |

Ces résultats montrent que l'adsorption des fibres ne varie pas entre 3 et 24 heures. Par ailleurs, la quantité d'OTA adsorbée augmente en fonction de la quantité de fibres présentes dans le milieu.

Les effets de durées de mise en contact inférieures à 24 heures sur le taux d'adsorption d'OTA (en %) par les fibres (20 g de fibres Realdyme® M par litre de milieu liquide modèle à pH 5,2 contenant 35 ng d'OTA/ml) sont reportés dans le Tableau II ci-après :

**TABLEAU II**

| Durée de la mise en contact (en minutes) | % d'OTA adsorbé |
|---|---|
| 0 | 0 |

(suite)

| Durée de la mise en contact (en minutes) | % d'OTA adsorbé |
|---|---|
| 5 | 21 |
| 15 | 20 |
| 45 | 25 |
| 90 | 29 |
| 169 | 28 |
| 360 | 30 |
| 1440 | 43 |

Ces résultats montrent que l'adsorption se produit très rapidement (entre 5 et 45 minutes environ) et que celle-ci se maintient au moins pendant toute la durée de l'expérience.

Les effets de la micronisation sur la quantité d'OTA adsorbée par des fibres Realdyme® M et de leur matière première non-micronisée sont reportés dans le Tableau III ci-après :

**TABLEAU III**

| | Quantité d'OTA adsorbée (%) | |
|---|---|---|
| Quantité de fibres (g/l) | Matière première non micronisées | Realdyme® M |
| **20** | 17% | 33% |
| **30** | 22 % | 37% |

Ces résultats montrent que la micronisation permet d'améliorer la capacité d'adsorption des fibres d'un facteur proche de deux.

La figure 1 annexée représente la capacité d'adsorption de trois fibres différentes (Realdyme® M : croix ; Realdyme® OF : ronds pleins et Realdyme® OM : triangles vides) en milieu DYP contenant une concentration initiale de 30 ng d'OTA/ml pour un volume initial de 25 ml et une durée de mise en contact de 45 minutes. Sur cette figure, le pourcentage d'OTA résiduelle est exprimé, pour chaque fibre, en fonction de la quantité de fibres en grammes par litre de milieu DYP.

Les résultats représentés sur la figure 1 montrent que même à des concentrations aussi faibles que 5 g de fibres par litre de milieu, on observe une bonne adsorption de l'OTA, en particulier avec les fibres Realdyme® M.

**EXEMPLE II: ETUDE DE L'EFFET DU pH SUR L'ADSORPTION DES MYCOTOXINES PAR DES FIBRES DE BLE**

Le pH du milieu est susceptible d'influencer l'adsorption de l'OTA sur les fibres car il influence la répartition des charges électriques sur les fibres, sur les toxines et dans le milieu. Au cours de la digestion, le pH du bol alimentaire diminue dans de larges proportions.

Afin d'étudier l'impact du pH sur la capacité d'adsorption des fibres, une expérience en milieu modèle a été réalisée, consistant en la mesure de l'adsorption avant et après une descente puis une remontée du pH.

**1) Protocole expérimental** :

Une quantité connue de fibres Realdyme® M correspondant à la concentration de 20 g de fibres/l est mélangée dans un tube stérile de 50 ml à 25 ml de milieu modèle DYP tel que décrit ci-dessus à l'exemple 1, préalablement contaminé à 50 ng d'OTA/ml au moyen d'une solution d'OTA dans de l'éthanol. Le pH du milieu est mesuré à 6.

Le contenu du tube est alors incubé, séparé, extrait, purifié et analysé comme décrit ci-dessus à l'exemple 1.

Parallèlement, le pH de ce même DYP est, dans deux autres tubes déjà garnis de fibres, abaissé jusqu'à une valeur de 2,2 par ajout d'acide lactique solide. Le contenu d'un des deux tubes est alors incubé, séparé, extrait, purifié et analysé comme décrit ci-dessus à l'exemple 1.

Le pH du milieu dans le deuxième tube est alors remonté à 4,8 par ajout de granules d'hydroxyde de sodium. Le contenu du tube est alors incubé, séparé, extrait, purifié et analysé comme dans l'exemple 1.

Chaque expérience est effectuée trois fois.

**2) Résultats :**

Les résultats obtenus sont reportés sur la figure 2 annexée qui représente l'évolution de l'adsorption de l'OTA (diminution de la quantité d'OTA dans le milieu DYP en % de la quantité initialement présente) sur les fibres après la descente et après la remontée du pH dans le milieu DYP. Sur cette figure, la barre noire correspond aux mesures effectuées à pH 6, la barre grise aux mesures effectuées à pH 2,2 et la barre blanche aux mesures effectuées après remontée du pH de 2,2 à 4,8.

Il est manifeste que plus le pH est bas, plus l'adsorption de l'OTA par les fibres est forte, atteignant même pour un pH de 2,2 un pourcentage d'adsorption de 82,4 %. Le relargage de la toxine lors de la remontée du pH ne semble pas être aussi fort que l'augmentation de l'adsorption lors de l'abaissement du pH.

La diminution du pH permet donc, pour une même quantité de fibres, d'augmenter de façon considérable la quantité d'OTA adsorbée par ces fibres.

## EXEMPLE III : MISE EN EVIDENCE DE L'ADSORPTION DES AFLATOXINES B1 PAR DES FIBRES VEGETALES INSOLUBLES

Une quantité déterminée de fibres végétales (Realdyme® : 20 g/l) est introduite dans un tube stérile de 50 ml et mélangée avec 25 ml de tampon phosphate à pH 7 (PBS), préalablement contaminé par les aflatoxines B1 (environ 8,5 ppb). Après homogénéisation manuelle pendant 30 secondes, le tube est placé à agiter à 90 tours par minute dans une chambre thermostatée à 25°C pendant 45 minutes. Un traitement contrôle sans adsorbant a servi de témoin.

A l'échéance, la suspension est centrifugée à 1830g pendant 10 minutes à 25°C, puis le culot est séparé du surnagent. L'essai est réalisé trois fois.

Les aflatoxines B1 (initiales et résiduelles) sont analysées par une méthode immunochimique ELISA de compétition directe à l'aide du test spécifique quantitatif à haute sensibilité vendu sous la dénomination commerciale Veratox® HS par la société Neogen Corporation (USA). Le protocole préconisé par le fournisseur de ce test a été utilisé.

Ce test immunochimique (ELISA) a été réalisé de manière suivante :

-   dépôt de 100 $\mu$l de conjugué dans chaque micropuits non tapissé d'anticorps ;
-   ajout de 100 $\mu$l de standard ou de 100 $\mu$l d'échantillon et mélange ;
-   prélèvement de tout le mélange et son dépôt dans un micropuits tapissé d'anticorps ;
-   incubation pendant 10 minutes à température ambiante ;
-   lavage cinq fois avec de l'eau désionisée ;
-   dépôt de 100 $\mu$l de substrat ;
-   incubation pendant 10 minutes à la température ambiante ;
-   ajout de 100 $\mu$l de solution "Red Stop" fournie avec le test pour arrêter la réaction substrat-enzyme.

En parallèle, on réalise la même expérience à pH 3 dans un milieu PBS (dont le pH a été ajusté à 3 avec une quantité suffisante d'acide lactique) ainsi qu'une courbe d'étalonnage avec des standards.

La densité optique des colorations est ensuite lue à une longueur d'onde de 620 nm à l'aide d'un lecteur de microplaque vendu sous la dénomination commerciale Labsystem Multiscan MCC/340-RS232C (Labsystems, Finlande).

La limite de détection et la limite de quantification de cette méthode analytique sont respectivement estimées à 3 et 10 ppt alors que le pourcentage de récupération est de 100 %.

Les résultats de cet essai sont présentés dans le Tableau IV ci-après :

**TABLEAU IV**

| Durée de mise en contact (min) | Aflatoxines B1 adsorbées à pH 7 (%) | Aflatoxines B1 adsorbées à pH 3 (%) |
|---|---|---|
| 0 | 0 | 0 |
| 5 | 66 | 72 |
| 25 | 68 | 69 |
| 45 | 68 | 70 |
| 120 | 67 | 74 |

Ces résultats montrent que les fibres Realdyme® permet d'adsorber une grande quantité d'Aflatoxines B1, et ce dès 5 minutes de contact, à pH neutre comme à pH acide.

## EXEMPLE IV : ETUDE DE L'EFFET DOSE-RFPONSE LORS DE L'ADSORPTION DE L'AFB1 PAR DES FIBRES DE BLE MICRONISEES OU NON MICRONISEES

Le but de cet exemple est de déterminer à partir de quelle dose les fibres de blé micronisées (REALDYME®) et non micronisées, adsorbent la même quantité d'AFB1.

L'expérience a été réalisée dans du tampon PBS à pH 3 (le pH étant ajusté avec de l'acide lactique). Le tampon PBS a initialement été contaminé à une teneur d'environ 8 ppb d'AFB1.

L'effet dose réponse-réponse a été évalué pour des doses de 0,5 %, 1 %, 2 %, 5 % et 10 % en poids de chacune des deux fibres étudiées ; le dosage de l'AFB1 étant réalisé comme décrit ci-dessus à l'exemple 3.

Les résultats obtenus sont représentés sur la figure 3 annexée sur laquelle l'adsorption d'AFB1, exprimée en pourcentage de réduction de la concentration d'AFB1 dans le surnageant par rapport à la concentration initialement présente, est fonction de la quantité de fibres utilisées (en % pondéral) ; les carré noirs correspondent aux fibres de blé micronisées, les losanges noirs correspondent aux fibres de blé non micronisées.

Ces résultats montrent que les fibres de blé micronisées sont nettement plus performantes vis-à-vis de l'adsorption de l'AFB1.

D'un point de vue commercial, il est intéressant de remarquer que la dose de 0,75 % de fibres de blé micronisées a le même effet que la dose de 5 % de la même fibre non micronisée. Ces quantités adsorbent, toutes deux, 50 % d'AFB1 du milieu PBS à pH 3 contaminé initialement à environ 8 ppb. Par conséquent, l'utilisation de fibres végétales micronisées présente un grand intérêt commercial dans la mesure où cela permet de diminuer la quantité de matière première nécessaire à l'adsorption d'une quantité de mycotoxines donnée.

## EXEMPLE V: MISE EN EVIDENCE DE L'EFFET DE L'ADMINISTRATION D'UNE COMPOSITION ALIMENTAIRE COMPRENANT DES FIBRES VEGETALES MICRONISEES SUR LA DIMINUTION DE LA BIODISPONIBILITE DE L'OTA CHEZ LE RAT

Cet exemple a été réalisé dans le but de montrer que les effets observés *in vitro* notamment à l'exemple 1 ci-dessus en terme d'adsorption des mycotoxines par des fibres végétales sont conservés *in vivo* et permettent de diminuer la biodisponibilité des mycotoxines après ingestion d'un aliment contaminé.

Plus particulièrement, le présent exemple vise, d'une part, à évaluer l'effet de ces adsorbants biologiques sur les performances de croissance et sur la quantité des matières fécales chez des rats exposés à la toxine *via* une alimentation naturellement contaminée. D'autre part, il vise à évaluer l'effet protecteur de ces fibres sur les teneurs en OTA dans le sang et les organes.

De façon spécifique, cet exemple vise à :

- tester l'impact de l'incorporation des fibres de blé micronisées dans l'alimentation naturellement contaminée par l'OTA sur la croissance et l'évolution de la masse corporelle des rats exposés à la toxine ;
- tester l'impact de l'incorporation des fibres de blé micronisées sur la quantité des matières fécales éliminées en présence de l'OTA ;
- évaluer la capacité des fibres de blé micronisées à diminuer la quantité d'OTA dans le plasma sanguin, les reins et le foie des rats naturellement exposés à l'OTA ;
- tester l'impact de l'incorporation des fibres de blé micronisées sur la teneur en OTA dans les matières fécales.

### I) Matériels et Méthodes

### 1) Produits

- Potato-extract, Dextrose/Glucose et Gélose (agar-agar) (poudres) vendus par la société Scharlan Chemie S. A., Barcelone, Espagne ;
- Peptone en poudre (Duchefa, Haarlem, Pays-Bas) ;
- Ochratoxine A (O-1877) (Sigma Chemical Co, St Louis, MO, Etats-Unis) ;
- Toluène pour analyse (Lab Scan, Dublin, Irlande) ;
- Chloroforme (grade analytique) (Lab Scan, Dublin, Irlande) ;
- Ether (Labscan, Dublin, Irlande) ;
- Méthanol grade HPLC (Acros Organics, Geel, Belgique) ;
- Acétonitrile grade HPLC (Lab Scan, Dublin, Irlande) ;
- Tampon phosphate : Phosphate Buffer Saline-PBS (NaCl 120 mmol/l, KCl 2,7 mmol/l, phosphate buffer 10 mmol/l ; pH 7,4) (Sigma Chemical Co, St Louis, MO, Etats-Unis) ;

- Bicarbonate de sodium (Merck, Darmstadt, Allemagne) ;
- Chlorure de sodium (Merck, Darmstadt, Allemagne) ;
- Eau Milli-Q Plus (Millipore, Molsheim, France) ;
- Acide acétique 99-100 % pour analyse (UCB, Bruxelles, Belgique) ;
- Acide orthophosphorique (grade analytique) (UCB, Bruxelles, Belgique) ;
- Bonbonne (gaz comprimé) d'azote et d'hélium (Air Liquide, Liège, Belgique) ;
- Tubes polypropylène (Falcon) stériles de 15 et 50 ml (Greiner-Labortechnik, Frickenhausen, Allemagne) ;
- Colonnes d'immunoaffinité Ochratest ® (Vicam, Watertown, MA, Etats-Unis) ;
- Filtre de type Millex®-HV GVHP 04700 (0,22 $\mu$m) (Millipore, Bedford, MA, Etats-Unis) ;
- Filtre Acrodisk ® 13 mm pour seringues avec membrane en Nylon 0,45 $\mu$m (Pall Gelman Laboratory, Karlstein, Allemagne).

### 2) Appareils

- Support Vac-Elut ® (Analytichem International, Harbour City, CA, Etats-Unis) ;
- Ultraturax ® T-25 basic (IKA-Werke, Janke und Kunkel GMBH & Co, Staufen, Allemagne) ;
- Evaporateur Rotavapor <R> (Büchl, Flawil, Suisse) ;
- Centrifugeuse Jouan CR (Jouan, Saint Nazaire, France) ;
- Spectrophotomètre OPI - 4 (Shimadzu, Kyoto, Japon) ;
- Autoclave (Fedegari Autoclavi, Spa-Albuzzano-Pv, Italie) ;
- Système de chromatographie liquide haute performance (HPLC) constitué de :

   * Pompe à régime isocratique: Perkin Elmer LC049 (Norwalk, CO, Etats-Unis), avec une boucle d'injection de 50 $\mu$l (Rheodyne, Cotati, CA, Etats-Unis) ;
   * Colonne : C 18 de 150 mm x 4.0 Hypersil ® BDS (phase inverse), porosité 3 $\mu$m, (Tracer Analytica, Barcelone, Espagne) ;
   * Détecteur à fluorescence RF 551 (Shimadzu, Kyoto, Japon) à lampe xénon 150 W réglé à $\lambda$excitation : 332 nm et $\lambda$émission : 462 nm ;
   * Intégrateur Spectra Physics SP4290 (San Jose, CA, Etats-Unis) ;
   * Phase mobile : acétonitrile : eau : acide acétique (450 : 540 : 10), filtrée sur membrane (0.25 $\mu$m) et dégazée à l'hélium (15 minutes) ;
   * Débit : 1 ml / minute ;
   * Pression : 2900-3000 psi.

### 3) Fibres végétales micronisées

Les fibres végétales testées sont les fibres de blé micronisées Realdyme® M (Realdyme, Garancières-en-Beauce, France) se présentant sous la forme de particules dont 90 % en poids ont une taille inférieure à 100 $\mu$m.

### 4) Production d'Ochratoxine A

L'alimentation des rats a été naturellement contaminée par l'OTA produite au sein du laboratoire de Biochimie de la Nutrition en étroite collaboration avec le laboratoire de mycologie de l'Unité de Microbiologie de l'Université Catholique de Liège (Belgique).

#### 4.1. Microorganisme

L'OTA a été produite en cultivant la souche de *Penicillium verrucosum* (code MUCL : CWL 44468 de la Mycothèque de l'Université de Louvain la Neuve, Belgique) sur des grains de blé.

#### 4.2. Préparation de l'inoculum

La souche a été revitalisée pendant une semaine par repiquage sur un milieu gélosé Potato-Dextrose-Agar (PDA) constitué d'un bouillon de potato-extract (0,4 %), de dextrose (2%) et d'agar (1,5 %). Le PDA a été préalablement stérilisé à l'autoclave (15 min à 121°C), coulé en pente et conservé à +4 °C au réfrigérateur. Les conidies produites sur PDA au bout de 10 jours de culture ont été décollées aseptiquement à l'aide d'une anse stérile et mises en suspension dans de l'eau peptonée (9 %) stérile. Deux ou trois dilutions successives de 1/10 ont été indispensables pour le comptage des conidies par examen direct au microscope. Une charge de la suspension de $10^4$ conidies/ml a été utilisée afin d'inoculer les grains de blé à la dose de 2 ml par 60 g.

### 4.3. Culture

La culture de la souche a été réalisée sur du blé tendre. Pour ce faire, l'humidité des graines de blé a été ajustée à 24-25% par addition d'un volume d'eau approprié. La culture a été réalisée dans des erlenmeyers de 250 ml contenant 60 g de grains de blé et stérilisés à l'autoclave (121°C pendant 20 min). Après l'inoculation de 2 ml de suspension de conidies dans des conditions aseptiques, les erlenmeyers ont été incubés à l'obscurité dans une chambre thermostatée à 22°C pendant 24 jours. A l'échéance, les échantillons ont été stérilisés comme précédemment, congelés et lyophilisés afin de faciliter le moulage. Une production totale de 2 kg de blé contaminé à raison de 22 $\mu$g d'OTA/g de blé a ainsi été obtenue.

### 4.4. Composition et préparation des régimes

L'aliment de base pour les rats a été fourni par Carfil (Parvan Service PVBA Carfil quality, Oud-Turnhout, Belgique) par sacs de 15 kg. Il est principalement constitué de protéines (21 %), de matières grasses (4,5 %), de cellulose (4 %), de cendres (7,0 %), de vitamine A (20 000 IE/UI/kg), de vitamine D3 (2000 IE/UI/kg) et de vitamine E (40 mg/kg).

Partant de cet aliment standard, les trois régimes figurant dans le tableau V ci-après ont été constitués :

<u>**TABLEAU V**</u>

| Régimes | Aliment de base (%) | Farine de blé (*) non contaminée (%) | Farine de blé contaminée à 22 $\mu$g d'OTA/g (%) | Fibres de blé micronisée |
|---|---|---|---|---|
| 1 | 88 | 12 | 0 | 0 |
| 2 | 88 | 2 | 10 | 0 |
| 3 | 88 | 0 | 10 | 2 |
| *: La teneur en OTA dans la farine de blé de départ a été déterminée (teneur OTA < Limite de Détection (LD)) | | | | |

Pour chacun de ces régimes, les différents constituants sont intimement mélangés et pressés sous forme de granulés cylindriques (diamètre = 5 mm). Un stock de 10,8 kg d'aliments a été ainsi constitué pour chaque régime. La teneur en OTA a été déterminée sur un échantillon de chaque régime prélevé à trois moments différents de l'expérimentation. Les 3 sous-échantillons sont mélangés et moulus.

### 5) Animaux et plan expérimental

Trente-six rats males de race Wistar/AF EOPS âgés de 9 semaines ont été fournis par le Centre d'élevage Janvier Laboratoire (Genest-St-Isle, France). A la réception, les rats ont été pesés (masse corporelle de départ variant entre 248,0 et 294,0 g avec une moyenne de 268,6 $\pm$ 10,5 g), et répartis dans des cages individuelles étiquetées. Ils ont été stabulés dans une animalerie climatisée à 22°C, soumise à un cycle de lumière-obscurité (alternatif) de 12 heures.

Après une familiarisation de 7 à 12 jours aux conditions de l'animalerie, les rats ont été répartis au hasard en 3 cohortes (n = 12).

### 5.1. Essais préliminaires

Les essais préliminaires ont été réalisés sur un échantillon de 4 rats supplémentaires de 3 mois, et visaient à la maîtrise des méthodes de prélèvements d'échantillons et d'analyse de l'OTA dans les plasma, foie et reins (par dopage). Ils visaient également la détermination des pourcentages de récupération de l'OTA pour les différentes méthodes analytiques mises au point au sein du laboratoire.

### 5.2. Plan expérimental

L'étude a été réalisée suivant un plan expérimental de cas-témoins : les 36 rats ont été répartis en 3 groupes, chaque groupe a reçu l'un des 3 régimes du tableau IV ci-dessus.

- Traitement 1 (Blanc) : Régime 1 non contaminé ;
- Traitement 2 (Témoin) : Régime 2 contaminé ;
- Traitement 3 : Régime 3 contaminé et incluant des fibres de blé micronisées (REALDYME ®).

Pour chacun des régimes, un rationnement systématique de 22 g d'aliments par jour et par rat a été délivré. Cette consommation reste juste en dessous de l'alimentation *ad libitum.* Les quantités d'aliments correspondant à une consommation de 48 heures ont été présentées aux rats tôt le matin, avant 8 heures (il est à noter que les rats n'ont pas été alimentés le jour du sacrifice). Les rats disposaient d'eau *ad libitum.*

Du point de vue hygiénique, la litière a été renouvelée systématiquement deux fois par semaine et les matières fécales éliminées ont été séparées et pesées.

Au cours du suivi, les rats ont été pesés tous les 3 jours jusqu'à l'échéance du 28ème jour. A cette échéance les rats ont été endormis à l'aide d'éther et sacrifiés par décapitation (à la guillotine) en vue des prélèvements de sang, du foie et des reins. Le sang a été immédiatement recueilli dans un tube de polypropylène de 15 ml, préalablement héparinisé. Après centrifugation à 1830 g pendant 20 min, le plasma sanguin (2,5 - 5 ml) a été récupéré et stocké à -20°C jusqu'à l'extraction de l'OTA. Les reins et le foie ont été pesés, congelés dans l'azote liquide (freeze-clamping) et immédiatement conservés à -80°C. Le reste de la carcasse a été conservé à -20°C.

## 6) Détermination de l'OTA dans les différentes matrices biologiques

### 6.1 Extraction de l'OTA dans les céréales et les granulés d'aliments

L'extraction de l'OTA des céréales et des granulés d'aliments est basée sur la technique (ISO FDIS 1541.2) du Comité Européen de Normalisation (CEN/TC 275, 1998) de l'Union Européenne. Le protocole utilisé est le suivant :

- Peser 50,0 g de farine dans un tube à centrifuger (préalablement nettoyé et séché) ;
- Ajouter 200 ml de chloroforme et 20 ml d'acide phosphorique (0,1 M, pH 3) ;
- Homogénéiser pendant 3 minutes à 13 500 tours par minutes (rpm) à l'aide d'un Ultraturax ® ;
- Centrifuger pendant 10 minutes à 820g à basse température (5-10°C) ;
- Récupérer toute la phase chloroformique ;
- Procéder à une deuxième extraction en répétant les points précédents ;
- Récupérer et combiner les deux phases chloroformiques ;
- Prélever 350 ml de la phase chloroformique récupérée ;
- Évaporer le chloroforme au rotavapor (30-40°C) ;
- Ajouter 100 ml de solution de carbonate de sodium (0,5 M, pH=9) au résidu d'évaporation ;
- Centrifuger pendant 10 minutes à 820g à basse température (5-10°C);
- Récupérer 20 ml de cette solution de bicarbonate et la purifier sur une colonne d'immunoaffinité conformément au protocole décrit au point 6.4 ci-après).

### 6.2. Extraction de l'OTA du plasma sanguin

- Prélever 2,5 ml de plasma ;
- Ajouter 20 ml de chloroforme et 10 ml d'acide phosphorique (0,1 M, pH 3) ;
- Homogénéiser pendant 3 minutes à l'aide d'un Vortex ;
- Centrifuger pendant 10 minutes à 820g à basse température (5-10°C) ;
- Récupérer toute la phase chloroformique à l'aide d'une pipette ;
- Procéder à une deuxième extraction en répétant les points précédents ;
- Récupérer toute la phase chloroformique à l'aide d'une pipette et combiner les deux phases chloroformiques ;
- Évaporer le chloroforme au rotavapor (30-40°C) ;
- Ajouter 20 ml de solution de carbonate de sodium (0,5 M, pH=9) au résidu d'évaporation ;
- Centrifuger pendant 10 minutes à 820g à basse température (5-10°C) ;
- Récupérer un volume de 15-20 ml de cette solution de bicarbonate et le purifier sur une colonne d'immunoaffinité selon le protocole décrit ci-après au point 6.4.

### 6.3. Extraction de l'OTA des reins, du foie et des fèces

Dans ce qui suit et sauf indications contraires, les instructions ci-après sont communes aux reins, au foie et aux fèces.

- Peser dans un tube à centrifuger :

    * Pour les reins : 2,5 g de reins,
    * Pour le foie : l'ensemble de l'organe,
    * Pour les fèces : 4 g de matières

- Pour les reins et le foie uniquement : ajouter 2 g de chlorure de sodium ;
- Ajouter 50 ml de chloroforme et 20 ml d'acide phosphorique (0,1 M, pH 3) ;
- Homogénéiser pendant 3 minutes à 13 500 rpm à l'aide d'un Ultraturax ® ;
- Centrifuger pendant 10 minutes à 820g à basse température (5-10°C) ;
- Récupérer la phase organique inférieure ;
- Procéder à une deuxième extraction en répétant les points précédents ;
- Évaporer le chloroforme au rotavapor, à une température comprise entre 30 et 40°C ;
- Pour les reins et les fèces uniquement : ajouter 100 ml de solution de carbonate de sodium (0,5 M, pH = 9) au résidu d'évaporation ;
- Pour le foie uniquement : ajouter 50 ml de solution de carbonate de sodium (0,5 M, pH = 9) au résidu d'évaporation ;
- Centrifuger pendant 10 minutes à 820g à basse température (5-10°C);
- Récupérer un volume de 15-20 ml de cette solution de bicarbonate et le purifier sur une colonne d'immunoaffinité comme décrit ci-après au point 6.4.

### 6.4. Purification sur colonne d'immunoaffinité (OchraTest®)

- Fixer la colonne sur un support Vac-Elut ® et la surmonter d'une seringue de 20 ml grâce à un adaptateur ;
- Conditionner la colonne d'immunoaffinité avec 20 ml de solution de PBS ;
- Passer 15-20 ml des solutions de bicarbonate sur la colonne à un débit de 1 à 2 ml/minute ;
- Laver la colonne avec 20 ml d'eau Milli-Q plus ;
- Récupérer l'OTA en éluant à l'aide de 2 ml de méthanol et 2 ml d'eau Milli-Q plus ;
- Faire passer 20 ml d'air à l'aide d'une seringue à travers la colonne en vue de récupérer l'entièreté de l'éluat.

### 6.5. Détection et quantification par HPLC

Cent $\mu$l d'extrait filtré ont été injectés afin de remplir complètement la boucle d'injection dont la capacité est de 50 $\mu$l La phase d'élution était composée d'un mélange acétonitrile/eau/acide acétique (540/450/10 ; v/v/v). La détection de l'OTA a été faite à 332 nm (excitation) et à 462 nm (émission). La concentration d'OTA a été déterminée par l'interpolation des hauteurs de pic sur la droite d'étalonnage au même temps de rétention.

Parallèlement, une droite d'étalonnage a été réalisée avec une solution stock diluée en des standards de 0,5 ; 1 ; 2 ; 3 ; 4 et 5 ng OTA /ml dans le mélange eau/méthanol (50/50 ; v/v). La droite d'étalonnage a été déterminée par la méthode des moindres carrés (non représentée).

### 7) Caractéristiques de performance des méthodes analytiques

*Limites de détection*

Tenant compte de toutes les procédures analytiques (extraction, purification et quantification), les limites de détection sont estimées à 10 ng/kg pour les céréales, 20 ng/l pour le plasma et 20 ng/kg pour les reins, le foie et les fèces.

### 8) Traitement des données et analyse statistique

La vitesse de croissance des rats a été calculée par différence entre deux masses corporelles consécutives, rapportée à la durée séparant les deux pesées.

L'effet induit par les régimes contaminés a été évalué par l'écart des résultats obtenus par rapport au Régime 1 (Blanc).

La procédure d'analyse non paramétrique a été utilisée compte tenu de l'hétérogénéité des données obtenues. Le test de Kruskal Wallis a permis de réaliser l'analyse de variance des différents traitements. Le test de Wilcoxon a été utilisé pour apprécier la différence entre les moyennes des masses corporelles des rats exposés aux régimes contaminés et celles des rats soumis au régime Blanc, au début et pendant les 4 semaines d'expérimentation alors que le test de Mann-Whitney a été exploité en vue d'apprécier la différence entre la moyenne d'un traitement et celle du Blanc ou du Témoin. Le test de corrélation de Spearman a été utilisé pour vérifier la consistance de la relation entre les teneurs d'OTA plasmatiques et rénales.

Les résultats sont exprimés sous forme de moyenne $\pm$ standard de déviation. Les données ont été analysées par le logiciel statistique SPSS version 10.0 (1999). Les différences sont considérées comme significatives à $p < 0,05$.

## II) Résultats

### 1) Consommation alimentaire et exposition des rats à l'OTA

Les résultats de dosage de l'OTA dans les trois régimes sont présentés dans le tableau VI ci-dessous :

**TABLEAU VI**

| Régimes | Traitements | Teneur en OTA dans le régime ($\mu$g/kg) | OTA totale ingérée * ($\mu$g) | Apport journalier en OTA ($\mu$g/kg de mc/jour) | |
|---|---|---|---|---|---|
| | | | | Moyenne $\pm$ SD | Etendue |
| Régime 1 | Blanc | 10,3 | 6,345 | 1,99 $\pm$ 0,40 | 2,57-1,23 |
| Régime 2 | Témoin | 2240,67 | 1380,252 | 438,42 $\pm$ 84,27 | 559,33 - 276,51 |
| Régime 3 | Fibres de blé | 2201,64 | 1356,210 | 433,52 $\pm$ 84,41 | 555,46-273,23 |
| * : Durée d'exposition = 28 jours, Consommation journalière de 22 g | | | | | |

Ces résultats montrent que l'aliment de base utilisé (Carfil) est légèrement contaminé en OTA (10,3 $\mu$g/kg) et que les incorporations de farine naturellement contaminée dans les deux autres rations ont permis d'obtenir une contamination moyenne de 2221,16 $\mu$g d'OTA/kg. Déjà, l'aliment de base servi à raison de 22 g par jour et par rat offre un apport journalier de 1,99 $\pm$ 0,4 $\mu$g/kg de masse corporelle (mc). Les deux autres rations servies à la même quantité exposent les rats à des apports journaliers moyens de 433,52 et 438,42 $\mu$g d'OTA/kg de mc selon le régime.

### 2) Impact de l'incorporation des fibres de blé micronisées dans les régimes sur la quantité de matières fécales éliminées par les rats exposés aux différents régimes

Les résultats obtenus sont reportés sur la figure 4 annexée. La figure 4 représente la quantité cumulée de matières fécales éliminées (en g) en fonction du temps pour chacun des régimes (barres grises : régime 1 ; barres noires : régime 2 et barres blanches : régime 3).

Ces résultats révèlent une différence significative dans les quantités de matières fécales excrétées par les rats soumis aux 3 régimes. Le test de comparaison multiple permet de distinguer trois classes ordonnées ci-après : rats soumis au régime 2 < rats soumis au régime 1 < rats soumis au régime 3.

L'administration répétitive de l'OTA *via* le régime 2 durant 28 jours, c'est-à-dire sans fibres de blé micronisées, diminue en permanence la quantité de matières fécales excrétées (figure 5). Par contre, l'ajout des fibres dans le régime augmente l'excrétion des matières fécales. En effet, ces fibres offrent une augmentation de l'ordre de 35 % par rapport au régime 2 et de l'ordre de 15 % par rapport au régime 1. L'augmentation de la masse de fèces est un des effets bien connus des fibres alimentaires.

### 3) Impact de l'incorporation de fibres de blé micronisées sur les concentrations en OTA dans le plasma sanguin, les reins, le foie et les fèces des rats exposés aux différents régimes

#### 3.1. Dosage de l'OTA Plasma

Le tableau VII récapitule ci-après les teneurs brutes d'OTA dans le plasma, ainsi que celles rapportées aux apports totaux en OTA durant la période d'exposition des rats aux régimes naturellement contaminés.

**TABLEAU VII**

| Régimes | Teneur en OTA dans le plasma sanguin | | Teneur en OTA dans le plasma sanguin rapportée à la quantité totale d'OTA ingérée | |
|---|---|---|---|---|
| | ng/ml | % de diminution [b] | ng/ml/$\mu$g d'OTA ingérée | % de diminution |
| 1 | 21,1 $\pm$ 39,0 | - | 3,3 $\pm$ 0,36 | - |
| 2 | 830,3 $\pm$ 411,9 | - | 0,60 $\pm$ 0,30 | - |
| 3 | 494,1 $\pm$ 186,3[a] | 40,5 | 0,37 $\pm$ 0,14 | 49,13 |
| [a] : Une différence significative est observée entre cette moyenne et celle des rats soumis au régime 2 témoin (p < 0,05) [b] : Pourcentage de la quantité d'OTA plasmatique des rats soumis au régime 3 rapportée à celle du régime 2 témoin. | | | | |

Ces résultats montrent que les fibres de blé micronisées, exercent une activité significative sur la diminution de la quantité d'OTA dans le plasma sanguin. Il est à noter que l'incorporation des fibres de blé micronisées à la dose de 2 % dans l'aliment permet de diminuer de 40,5 % la concentration d'OTA dans le plasma. Cette aptitude est aussi remarquée lorsque les teneurs sont rapportées aux quantités totales d'OTA ingérées pendant toute la période de l'expérimentation.

Par conséquent, l'incorporation de fibres de blé micronisées dans un produit alimentaire permet de diminuer significativement la biodisponibilité de l'OTA.

### 3.2. Dosage de l'OTA dans les reins

Les teneurs d'OTA brutes rénales et celles rapportées aux apports totaux en OTA durant la période d'exposition des rats aux régimes naturellement contaminés sont présentées dans le tableau VIII ci-après :

**TABLEAU VIII**

| Régimes | Teneur en OTA dans les reins | | Teneur en OTA dans les reins rapportée à la quantité totale d'OTA ingérée | |
|---|---|---|---|---|
| | ng/g | % de diminution [b] | ng/g/$\mu$g d'OTA ingérée | % de diminution |
| 1 | 1,39 $\pm$ 1,66 | - | 0,2 $\pm$ 0,259 | - |
| 2 | 79,38 $\pm$ 31,4 | - | 0,057 $\pm$ 0,023 | - |
| 3 | 57,07 $\pm$ 42,5[a] | 28,11 | 0,043 $\pm$ 0,032 | 24,56 |
| [a] : Une différence significative est observée entre cette moyenne et celle des rats soumis au régime 2 témoin (p < 0,05). [b] : Pourcentage de la quantité d'OTA rénale des rats soumis au régime 3 rapportée à celle du régime 2 témoin. | | | | |

Ces résultats montrent que les fibres de blé exercent aussi une activité significative sur la diminution de la concentration de l'OTA dans les reins. Il est en effet à noter que l'incorporation de fibres de blé micronisées à la dose de 2 % dans l'aliment permet de diminuer de 28,11 % la concentration en OTA dans les reins.

La teneur en OTA dans les reins présente une relation linéaire avec la concentration d'OTA plasmatiques, illustrée par l'équation de régression suivante :

$$OTA_{reins} = (15,45 \pm 6,512) + (0,081 \pm 0,02)*OTA_{plasma}\ (R^2 = 0,620\ ;\ p < 0,0001).$$

Si le rein est l'organe d'accumulation de l'OTA, les teneurs qu'on y retrouve sont de bons indicateurs de la contamination à laquelle l'organisme est réellement soumis pendant une période relativement longue.

### 3.3. Dosage de l'OTA dans le foie

Les teneurs d'OTA brutes hépatiques et celles rapportées aux apports totaux en OTA durant la période d'exposition des rats aux régimes naturellement contaminés sont présentées dans le Tableau IX ci-après :

**TABLEAU IX**

| Régimes | Teneur en OTA dans le foie | | Teneur en OTA dans le foie rapportée à la quantité totale d'OTA ingérée | |
|---|---|---|---|---|
| | ng/g | % de diminution | ng/g/$\mu$g d'OTA [b] ingérée | % de diminution [b] |
| 1 | 1,43 $\pm$ 3,5 | - | 0,24 $\pm$ 0,56 | - |
| 2 | 73,68 $\pm$ 31,43 | - | 0,0483 $\pm$ 0,0515 | - |
| 3 | 45,13 $\pm$ 17,38 [a] | 38,58 | 0,0250 $\pm$ 0,0452 [a] | 40,05 |

[a] : Une différence significative est observée entre cette moyenne et celle des rats soumis au régime 2 Témoin ($p < 0,05$).
[b] : Pourcentage de la concentration d'OTA hépatique des rats soumis au régime 3 rapportée à celle du régime 2 Témoin.

Ces résultats montrent que les fibres de blé exercent une activité significative sur la diminution de la concentration de l'OTA dans le foie. L'incorporation de fibres de blé micronisées à la dose de 2 % dans l'aliment permet de diminuer de 38,58 % la concentration en OTA dans le foie.

**3.4. Dosage de l'OTA dans les fèces**

En fonction de l'efficacité de l'absorption gastro-intestinale et du métabolisme hépatique, les mycotoxines et leurs métabolites sont préférentiellement excrétés par les voies urinaire et fécale. Les quantités d'OTA relevées dans les fèces sont données dans le Tableau X ci-après :

**TABLEAU X**

| Régimes | Quantité d'OTA dans les fèces récoltés pendant la semaine 1 | | Quantité d'OTA dans les fèces récoltés pendant la semaine 4 | |
|---|---|---|---|---|
| | $\mu$g | % d'augmentation [b] | $\mu$g | % d'augmentation [b] |
| 1 | 0,046 $\pm$ 0,0004 | - | 0,098 $\pm$ 0,0016 | - |
| 2 | 35,27 $\pm$ 0,12 | - | 43,47 $\pm$ 0,17 | - |
| 3 | 58,41 $\pm$ 0,17[a] | 65,60 | 82,94 $\pm$ 0,13[a] | 90,80 |

[a] : Une différence significative est observée entre cette moyenne et celle des rats soumis au régime 2 Témoin ($p < 0,05$).
[b] : Pourcentage de la quantité d'OTA dans les matières fécales des rats soumis au régime 3 rapportée à celle du régime 2 Témoin.

Le dosage de l'OTA dans les matières fécales démontre l'effet positif des fibres alimentaires dans l'adsorption de l'OTA. En effet, d'après les résultats obtenus (Tableau X), l'addition des fibres biologiques dans la ration alimentaire a permis d'augmenter la quantité de l'OTA éliminée. Cette augmentation est de 65,60 % durant la première semaine et de 90,80 % pour la quatrième semaine.

Les teneurs d'OTA brutes dans les matières fécales des semaines expérimentales 1 et 4 et celles rapportées aux apports totaux en OTA durant les périodes correspondantes d'exposition des rats aux régimes naturellement contaminés sont présentées dans les tableaux XI et XII ci-après. Les valeurs obtenues montrent une fois de plus que les fibres de blé micronisées exercent une activité d'adsorption vis-à-vis de l'OTA.

**TABLEAU XI**

| Régimes | Teneur en OTA dans les fèces (Semaine 1) | | Teneur en OTA dans les fèces (Semaine 4) | |
|---|---|---|---|---|
| | $\mu$g/g | % d'augmentation [b] | $\mu$g/g | % d'augmentation [b] |
| 1 | 0,0014 $\pm$ 0,0004 | - | 0,003 $\pm$ 0,0016 | - |
| 2 | 1,251 $\pm$ 0,11 | - | 1,68 $\pm$ 0,168 | - |

(suite)

| Régimes | Teneur en OTA dans les fèces (Semaine 1) | | Teneur en OTA dans les fèces (Semaine 4) | |
|---|---|---|---|---|
| | $\mu$g/g | % d'augmentation [b] | $\mu$g/g | % d'augmentation [b] |
| 3 | 1,75 ± 0,17[a] | 40,20 | 2,55 ± 0 ,13[a] | 51,9 |
| [a] : Une différence significative est observée entre cette moyenne et celle des rats soumis au régime 2 Témoin ($p < 0,05$)<br>[b] : Pourcentage de la concentration d'OTA dans les fèces des rats soumis au régime 3 rapportée à celle du régime 2 Témoin. | | | | |

**TABLEAU XII**

| Régimes | Teneur en OTA dans les fèces rapportée à la quantité totale d'OTA ingérée (Semaine 1) | | Teneur en OTA dans les fèces rapportée à la quantité totale d'OTA ingérée (Semaine 4) | |
|---|---|---|---|---|
| | ng/g/$\mu$g d'OTA ingérée | % d'augmentation[b] | ng/g/$\mu$g d'OTA ingérée | % d'augmentation[b] |
| 1 | 0,86 ± 0,08 | - | 2,01 ± 0,34 | - |
| 2 | 3,62 ± 0,65 | - | 4,85 ± 0,14 | - |
| 3 | 5,15 ± 0,91 [a] | 42,27 | 7,48 ± 0,13 [a] | 54,23 |
| [a] : Une différence significative est observée entre cette moyenne et celle des rats soumis au régime 2 Témoin ($p < 0,05$)<br>[b] : Pourcentage de la concentration d'OTA dans les fèces des rats soumis au régime 3 rapportée à celle du régime 2 Témoin. | | | | |

Les résultats expérimentaux obtenus sont donc particulièrement significatifs de l'effet des fibres végétales micronisées sur la diminution de la biodisponibilité de l'OTA après ingestion d'un aliment contaminé.

L'ensemble des ces résultats montrent par conséquent que les fibres végétales micronisées adsorbent et retiennent les mycotoxines non seulement dans des milieux liquides modèles mais également dans le chyme gastro-intestinal des animaux. La biodisponibilité des mycotoxines est ainsi diminuée.

Ces fibres sont des éléments naturels provenant de céréales, ce qui présente un avantage pour leur utilisation en alimentation animale ou humaine.

**Revendications**

1. Utilisation de fibres végétales micronisées essentiellement insolubles se présentant sous la forme de microparticules dont au moins 90 % en poids présentent une taille inférieure à 700 $\mu$m à titre d'ingrédient dans la préparation d'une composition alimentaire destinée à diminuer la biodisponibilité des mycotoxines chez l'homme ou l'animal lors de l'ingestion d'un aliment susceptible d'être contaminé par lesdites mycotoxines.

2. Utilisation selon la revendication 1, **caractérisée par le fait que** les fibres végétales micronisées se présentent sous la forme de microparticules dont au moins 90 % en poids présentent une taille inférieure ou égale à 400 $\mu$m.

3. Utilisation selon la revendication 2, **caractérisée par le fait que** les fibres végétales micronisées se présentent sous la forme de microparticules dont au moins 90 % en poids présentent une taille comprise entre 2 $\mu$m et 200 $\mu$m inclusivement.

4. Utilisation selon l'une quelconque des revendications précédentes, **caractérisée par le fait que** ladite composition alimentaire est destinée à diminuer la biodisponibilité des mycotoxines hydrophobes.

5. Utilisation selon l'une quelconque des revendications précédentes, **caractérisée par le fait que** les fibres végétales sont choisies parmi les fibres issues :

   - de végétaux alimentaires choisis parmi les céréales, les légumineuses, les plantes potagères et les fruits,
   - de végétaux utilisés par l'industrie du papier choisis parmi les arbres, la canne à sucre, le bambou et la paille de céréale.

**6.** Utilisation selon la revendication 5, **caractérisée par le fait que** les fibres végétales issues de céréales sont choisies parmi les fibres de blé, d'orge, d'avoine, de maïs, de millet, de riz, de seigle, de sorgho, et leurs équivalents maltés.

**7.** Utilisation selon la revendication 5, **caractérisée par le fait que** les fibres issues de végétaux alimentaires, autres que les céréales, sont choisies parmi les fibres issues des pommes, des poires, des grains de raisins, des graines de lupin et de soja, des tomates, des pois et du café.

**8.** Utilisation selon l'une quelconque des revendications 1 à 6, **caractérisée par le fait que** la composition alimentaire est destinée à diminuer la biodisponibilité de l'Ochratoxine A, des aflatoxines, de la Fumonisine et/ou du déoxynivalénol, et que les fibres végétales micronisées sont choisies parmi les fibres de blé, les fibres d'avoine et leurs mélanges.

**9.** Utilisation selon la revendication 8, **caractérisée par le fait que** la composition alimentaire est destinée à diminuer la biodisponibilité de l'Ochratoxine A et que les fibres végétales sont des fibres de blé micronisées se présentant sous la forme de microparticules dont 90 % en poids présentent une taille inférieure ou égale à 100 $\mu$m.

**10.** Utilisation selon l'une quelconque des revendications précédentes, **caractérisée par le fait que** la composition alimentaire se présente sous la forme d'un complément alimentaire et que la quantité de fibres végétales micronisées au sein dudit complément représente jusqu'à 100 % en poids du poids total dudit complément.

**11.** Utilisation selon la revendication 10, **caractérisée par le fait que** la quantité de fibres végétales micronisées au sein dudit complément est comprise entre 80 et 100 % en poids du poids total dudit complément..

**12.** Utilisation selon l'une quelconque des revendications 1 à 8, **caractérisée par le fait que** la composition alimentaire est destinée à l'alimentation humaine et qu'elle se présente sous la forme d'un ingrédient alimentaire à ajouter au cours de la fabrication d'un produit alimentaire à raison de 0,05 à 20 % en poids par rapport au poids total dudit produit alimentaire.

**13.** Utilisation selon l'une quelconque des revendications 1 à 8, **caractérisée par le fait que** la composition alimentaire est destinée à l'alimentation animale et qu'elle se présente sous la forme d'une matière première à ajouter à la ration alimentaire journalière qui est donnée aux animaux domestiques ou d'élevage ou à incorporer à titre d'ingrédient au cours de la fabrication d'un aliment complet pour animaux domestiques ou d'élevage à raison de 0,05 à 10 % en poids par rapport au poids total de la ration alimentaire ou de l'aliment complet.

**Claims**

**1.** The use of essentially insoluble micronized plant fibers in the form of microparticles, at least 90% by weight of which are less than 700 $\mu$m in size, as an ingredient in the preparation of a nutritional composition for reducing mycotoxin bioavailability in humans or animals when a food liable to be contaminated with said mycotoxins is ingested.

**2.** The use as claimed in claim 1, **characterized in that** the micronized plant fibers are in the form of microparticles, at least 90% by weight of which are less than or equal to 400 $\mu$m in size.

**3.** The use as claimed in claim 2, **characterized in that** the micronized plant fibers are in the form of microparticles, at least 90% by weight of which are between 2 $\mu$m and 200 $\mu$m, inclusive, in size.

**4.** The use as claimed in any one of the preceding claims, **characterized in that** said nutritional composition is for reducing the bioavailability of hydrophobic mycotoxins.

**5.** The use as claimed in any one of the preceding claims, **characterized in that** the plant fibers are chosen from fibers derived:

- from nutritional plants chosen from cereals, leguminous plants, edible plants and fruits,
- from plants used by the paper industry, chosen from trees, sugar cane, bamboo and cereal straw.

6. The use as claimed in claim 5, **characterized in that** the plant fibers derived from cereals are chosen from wheat, barley, oat, maize, millet, rice, rye and sorghum fibers, and malted equivalents thereof.

7. The use as claimed in claim 5, **characterized in that** the fibers derived from nutritional plants, other than cereals, are chosen from fibers derived from apples, pears, grapeseeds, lupin and soya seeds, tomatoes, peas and coffee.

8. The use as claimed in any one of claims 1 to 6, **characterized in that** the nutritional composition is for reducing the bioavailability of ochratoxin A, aflatoxins, fumonisin and/or deoxynivalenol, and that the micronized plant fibers are chosen from wheat fibers and oat fibers, and mixtures thereof.

9. The use as claimed in claim 8, **characterized in that** the nutritional composition is for reducing ochratoxin A bioavailability, and that the plant fibers are micronized wheat fibers in the form of microparticles, 90% by weight of which are less than or equal to 100 $\mu$m in size.

10. The use as claimed in any one of the preceding claims, **characterized in that** the nutritional composition is in the form of a food supplement, and that the amount of micronized plant fibers in said supplement represents up to 100% by weight of the total weight of said supplement.

11. The use as claimed in claim 10, **characterized in that** the amount of micronized plant fibers in said supplement is between 80% and 100% by weight of the total weight of said supplement.

12. The use as claimed in any one of claims 1 to 8, **characterized in that** the nutritional composition is intended for human nutrition, and that it is in the form of a nutritional ingredient to be added during the manufacture of a food product at a rate of from 0.05% to 20% by weight relative to the total weight of said food product.

13. The use as claimed in any one of claims 1 to 8, **characterized in that** the nutritional composition is intended for animal nutrition, and that it is in the form of a starting material to be added to the daily food intake which is given to domestic or breeding animals, or to be incorporated, as an ingredient, during the manufacture of a complete food for domestic or breeding animals at a rate of from 0.05% to 10% by weight relative to the total weight of the food intake or of the complete food.

**Patentansprüche**

1. Verwendung von im Wesentlichen unlöslichen, mikronisierten Pflanzenfasern in Form von Mikropartikeln, von denen mindestens 90 Gew.-% kleiner als 700 $\mu$m groß sind, als Bestandteil in einer Zubereitung einer Nahrungsmittelzusammensetzung, die dazu bestimmt ist, die Bioverfügbarkeit von Mykotoxinen bei Menschen oder Tieren während der Nahrungsaufnahme eines Nahrungsmittels, das mit Mykotoxinen verseucht sein kann, zu verringern.

2. Verwendung nach Anspruch 1, **dadurch gekennzeichnet, dass** die mikronisierten Pflanzenfasern in Form von Mikropartikeln vorliegen, von denen mindestens 90 Gew.-% kleiner oder gleich 400 $\mu$m groß sind.

3. Verwendung nach Anspruch 2, **dadurch gekennzeichnet, dass** die mikronisierten Pflanzenfasern in Form von Mikropartikeln vorliegen, von denen mindestens 90 Gew.-% zwischen 2 $\mu$m und 200 $\mu$m, jeweils eingeschlossen, groß sind.

4. Verwendung nach irgendeinem der vorhergehenden Ansprüche, **dadurch gekennzeichnet, dass** die Nahrungsmittelzusammensetzung dazu bestimmt ist, die Bioverfügbarkeit von hydrophoben Mykotoxinen zu verringern.

5. Verwendung nach irgendeinem der vorhergehenden Ansprüche, **dadurch gekennzeichnet, dass** die Pflanzenfasern ausgewählt sind aus Fasern, die von:

- Nahrungsmittelpflanzen, die ausgewählt sind aus Getreidesorten, Hülsenfrüchten, Gemüsepflanzen und Früchten,
- in der Papierindustrie verwendeten Pflanzen, die ausgewählt sind aus Bäumen, Zuckerrohr, Bambus und Getreidestroh.

stammen.

6. Verwendung nach Anspruch 5, **dadurch gekennzeichnet, dass** die von Getreidesorten stammenden Pflanzenfasern ausgewählt sind aus Fasern von Weizen, Gerste, Hafer, Mais, Hirse, Reis, Roggen, Sorghum und den entsprechend mit Malz versetzten Fasern.

7. Verwendung nach Anspruch 5, **dadurch gekennzeichnet, dass** die von Nahrungsmittelpflanzen, anderen als Getreidesorten, stammenden Fasern, ausgewählt sind aus Fasern, die von Äpfeln, Birnen, Beeren, Lupinen- und Sojakörnern, Tomaten, Erbsen und Kaffee stammen.

8. Verwendung nach irgendeinem der Ansprüche 1 bis 6, **dadurch gekennzeichnet, dass** die Nahrungsmittelzusammensetzung dazu bestimmt ist, die Bioverfügbarkeit von Ochratoxin A, Aflatoxinen, Fumonisin und/oder Desoxynivalenol zu verringern, und die mikronisierten Pflanzenfasern aus Weizenfasern, Haferfasern und deren Mischungen ausgewählt sind.

9. Verwendung nach Anspruch 8, **dadurch gekennzeichnet, dass** die Nahrungsmittelzusammensetzung dazu bestimmt ist, die Bioverfügbarkeit von Ochratoxin A zu verringern, und die Pflanzenfasern mikronisierte Weizenfasern in Form von Mikropartikeln sind, von denen 90 Gew.-% kleiner oder gleich 100 $\mu$m groß sind.

10. Verwendung nach irgendeinem der vorhergehenden Ansprüche, **dadurch gekennzeichnet, dass** die Nahrungsmittelzusammensetzung in Form eines Nahrungsmittelzusatzes vorliegt und die Menge an mikronisierten Pflanzenfasern in dem Zusatz bis zu 100 Gew.-% des Gesamtgewichts des Zusatzes ausmacht.

11. Verwendung nach Anspruch 10, **dadurch gekennzeichnet, dass** die Menge an mikronisierten Pflanzenfasern in dem Zusatz zwischen 80 und 100 Gew.-% des Gesamtgewichts des Zusatzes ausmacht.

12. Verwendung nach irgendeinem der Ansprüche 1 bis 8 **dadurch gekennzeichnet, dass** die Nahrungsmittelzusammensetzung für die Nahrungsversorgung von Menschen bestimmt ist und in Form eines Nahrungsmittelbestandteils in einem Anteil von 0,05 bis 20 Gew.-%, bezogen auf das Gesamtgewicht des Nahrungsmittelprodukts, während der Herstellung eines Nahrungsmittelprodukts beigefügt wird.

13. Verwendung nach irgendeinem der Ansprüche 1 bis 8, **dadurch gekennzeichnet, dass** die Nahrungsmittelzusammensetzung für die Nahrungsversorgung von Tieren bestimmt ist und in Form eines Rohstoffs in einem Anteil von 0,05 bis 10 Gew.-%; bezogen auf das Gesamtgewicht einer Nahrungsration oder einer Komplettnahrung, der täglichen Nahrungsration, die Haustieren oder Zuchttieren gegeben wird, beigefügt oder während der Herstellung der Komplettnahrung für Hautiere oder Zuchttiere als Bestandteil darin eingeschlossen wird.

FIGURE 1

FIGURE 2

FIGURE 3

FIGURE 4

FIGURE 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- DE 3810004

- FR 2433910

**Littérature non-brevet citée dans la description**

- **ABARCA ML. et al.** *J. Food Prot.,* 2001, vol. 64 (6), 903-906
- **WALKER R.** *Adv. Exp. Med. Biol.,* 2002, vol. 504, 249-255
- **PITTET A.** *Rev. Méd. Vét.,* 1998, vol. 149, 479-492
- **HUWIG A. et al.** *Tox. Letters,* 2001, vol. 122, 179-188
- **DIAZ D. E. et al.** *J. Dairy Sci.,* 1999, vol. 82, 838
- **GALVANO et al.** *J. Food Prot.,* 1997, vol. 60, 985-991
- **RAMOS A. J. ; HEMANDEZ E.** *Animal Feed Science and Technology,* 1997, vol. 62, 263-269

- Nutritional approaches to reduce the impact of mycotoxins. **PIVA A. ; GALVANO F.** Proceedings of Alltech's 15th Annual Symposium. Nottingham University Press, 1999, 381-400
- *Monatsh Veterinarmed,* 1994, vol. 49, 175-181
- *Poultry Science,* 1993, vol. 72, 282-288
- In vitro binding mycotoxins by adsorbents does not always translate into in vivo efficacy. **LEDOUX D. R. et al.** Mycotoxins and phycotoxins in perspective at the turn of the millennium. Proceedings of the Xth International IUPAC Symposium in Mycotoxins and Phycotoxins. 2001, 279-287
- **SANTIN E. et al.** *J. Appl. Poultry Res.,* 2002, vol. 11 (1), 22-28